# EUROPEAN PATENT APPLICATION

(11) **EP 2 226 024 A2**
(43) Date of publication of application: **08.09.2010**
(21) Application number: 10250400.8
(22) Date of filing: 05.03.2010
(51) Int. Cl.: A61B 17/34

(54) **Methods and devices for providing access into a body cavity**

(30) Priority: 06.03.2009 US 399482
(71) Applicant: Ethicon Endo-Surgery, Inc., Cincinnati, OH 45242 (US)
(72) Inventor: Weisenburgh, II, William B., Maineville, Ohio 45039 (US); Hess, Christopher J., Cincinnati, Ohio 45206 (US); Murray, Michael A., Bellevue, Kentucky 41073 (US); Holcomb, Matthew, Lebanon, Ohio 45036 (US); Gill, Robert P., Mason, Ohio 45040 (US)
(74) Representative: Tunstall, Christopher Stephen

(57) **Abstract**

A surgical access device (10), comprising a retractor (18) having a working channel (18a) extending therethrough for forming a pathway through tissue into a body cavity; and a housing (12) having a plurality of sealing ports (22a,22b,22c) configured to releasably mate to the retractor (18) in only one predetermined motivational orientation; and wherein the housing (12) has a plurality of mating elements (46a,46b,46c) that are configured to releasably engage the retractor (18) to mate the housing to the retractor (18), and wherein at least one (46c) of the plurality of mating elements differs from another one of the plurality of mating elements.

## Description

The present invention relates to methods and devices for providing surgical access into a body cavity.

### BACKGROUND OF THE INVENTION

Abdominal laparoscopic surgery gained popularity in the late 1980s, when benefits of laparoscopic removal of the gallbladder over traditional (open) operation became evident. Reduced postoperative recovery time, markedly decreased post-operative pain and wound infection, and improved cosmetic outcome are well established benefits of laparoscopic surgery, derived mainly from the ability of laparoscopic surgeons to perform an operation utilizing smaller incisions of the body cavity wall.

Laparoscopic procedures generally involve insufflation of the abdominal cavity with CO₂ gas to a pressure of around 15 mm Hg. The abdominal wall is pierced and a 5-10 mm in diameter straight tubular cannula or trocar sleeve is then inserted into the abdominal cavity. A laparoscopic telescope connected to an operating room monitor is used to visualize the operative field, and is placed through a the trocar sleeve. Laparoscopic instruments (graspers, dissectors, scissors, retractors, etc.) are placed through two or more additional trocar sleeves for the manipulations by the surgeon and surgical assistant(s).

Recently, so-called "mini-laparoscopy" has been introduced utilizing 2-3 mm diameter straight trocar sleeves and laparoscopic instruments. When successful, mini-laparoscopy allows further reduction of abdominal wall trauma and improved cosmesis. Instruments used for mini-laparoscopic procedures are, however, generally more expensive and fragile. Because of their performance limitations, due to their smaller diameter (weak suction-irrigation system, poor durability, decreased video quality), mini-laparoscopic instruments can generally be used only on selected patients with favorable anatomy (thin cavity wall, few adhesions, minimal inflammation, etc.). These patients represent a small percentage of patients requiring laparoscopic procedures. In addition, smaller 2-3 mm incisions may still cause undesirable cosmetic outcomes and wound complications (bleeding, infection, pain, keloid formation, etc.).

Since the benefits of smaller and fewer body cavity incisions are proven, it would be desirable to perform an operation utilizing only a single incision. An umbilicus is well-hidden and the thinnest and least vascularized area of the abdominal wall. The umbilicus is generally a preferred choice of abdominal cavity entry in laparoscopic procedures. An umbilical incision can be easily enlarged (in order to eviscerate a larger specimen) without significantly compromising cosmesis and without increasing the chances of wound complications.

Thus, there is a need for instruments and trocar systems which allow laparoscopic procedures to be performed entirely through the umbilicus or a surgical port located elsewhere while at the same time allowing adjustment of instrument position during the surgical procedure.

### SUMMARY OF THE INVENTION

The present invention generally provides methods and devices for providing surgical access into a body cavity. In one embodiment, a surgical access device is provided that includes a retractor having a working channel extending therethrough for forming a pathway through tissue into a body cavity, and a housing having a plurality of sealing ports configured to receive an instrument therethrough and into the working channel. The housing is configured to releasably mate to the retractor in only one predetermined rotational orientation.

In some embodiments the device can include an alignment mechanism, a plurality of mating elements, and/or a flexible shield. The alignment mechanism can be formed on at least one of the housing and the retractor and be configured to indicate alignment of the housing and the retractor in the predetermined rotational orientation. The alignment mechanism can vary in any number of ways. For example, the alignment mechanism can include a first protrusion extending laterally outward from a sidewall of the housing and configured to align with a second protrusion extending laterally outward from a sidewall of the retractor.

The housing can have a plurality of mating elements that are configured to releasably engage the retractor to mate the housing to the retractor. At least one of the plurality of mating elements can differ from another one of the plurality of mating elements. The mating elements can have any number of variations. For example, the mating elements can rotatably mate the housing to the retractor. As another example, the plurality of mating elements can include a plurality of feet extending distally from the housing and configured to engage a plurality of proximally-facing openings formed in the retractor. A proximal portion of the retractor can have the plurality of proximally-facing openings formed therein, and the plurality of feet can be configured to engage the proximal portion of the retractor when the housing is rotated relative to the retractor from the predetermined rotational orientation. For another example, at least one of the plurality of proximally-facing openings formed in the retractor can have a longitudinal length that differs from at least one other of the plurality of proximally-facing openings. For yet another example, each of the plurality of proximally-facing openings formed in the retractor can have a size that differs from one another, and each of the plurality of feet can have a size corresponding to one of the plurality of openings.

A flexible shield can be disposed within the retractor when the housing is mated to the retractor and can be configured to protect the retractor from damage caused by insertion of a surgical instrument through the retractor. The flexible shield can include a plurality of flexible strips extending distally from the housing.

The device can vary in any other number of ways. For example, the housing can be configured to rotate relative to the retractor from an initial mating position, in which the housing is freely releasable from the retractor, to a second rotated position, in which the housing is prevented from disengagement from the retractor. For another example, each sealing port can include an opening formed through the housing and have at least one sealing element disposed therein. The at least one sealing element can be configured to form at least one of a seal around an instrument inserted therethrough and a channel seal configured to form a seal when no instrument is inserted therethrough. For still another example, the device can include a plurality of housings, each housing having a plurality of sealing ports formed therein. Each housing can be interchangeably, releasably matable to the retractor.

In another embodiment, a surgical access device is provided that includes a retractor having an opening extending therethrough for forming a pathway through tissue into a body cavity, and a housing rotatably coupled to the retractor and having a plurality of sealing ports in communication with the opening in the retractor. Each of the sealing ports has a sealing element fixedly disposed therein, and at least one of the sealing elements has a linear distal opening extending tangential to a perimeter of the opening in the retractor and at least one other of the sealing elements has a linear distal opening extending perpendicular to the perimeter of the opening in the retractor. In some embodiments, the housing can have a plurality of mating elements that are configured to releasably engage the retractor to mate the housing to the retractor. At least one of the plurality of mating elements can differ from another one of the plurality of mating elements. The device can also include an alignment mechanism formed on at least one of the housing and the retractor and configured to indicate a single rotational orientation of the housing relative to the retractor in which the mating elements can releasably engage the retractor. The device can have any number of variations. For example, two of the sealing elements can have linear distal openings tangent to the perimeter of the opening. For another example, the housing can be configured to be releasably mated to the retractor. For yet another example, the device can include a plurality of flexible shields extending from the housing and configured to distally extend into the opening of the retractor when the housing is coupled to the retractor. The linear distal opening extending tangential to the perimeter of the opening in the retractor and the linear distal opening extending perpendicular to the perimeter of the opening in the retractor can each extend parallel to a planar surface of at least one of the flexible shields.

In yet another embodiment, a surgical access device includes a proximal portion having a plurality of access ports formed therein for receiving surgical instruments therethrough, a distal portion removably coupled to the proximal portion and having an opening extending therethrough for forming a pathway through tissue into a body cavity, and a plurality of flexible shields extending from the proximal portion and configured to extend into the opening of the distal portion, each of the flexible shields being located adjacent to one of the access ports. In some embodiments, each of the access ports can have a sealing element disposed therein that is configured to form a seal when no instrument is inserted therethrough. Each of the sealing elements can have an elongate slot formed at a distal end thereof, and a plane of each of the flexible shields can be parallel to at least one of the elongate slots. Each sealing element can be in a substantially fixed position relative to the proximal portion, and when the proximal portion is coupled to the distal portion at least one of the elongate slots can extend tangential to a perimeter of the opening in the distal portion and at least one of the elongate slots can extend perpendicular to a perimeter of the opening in the distal portion. At least one of the elongate slots can extend tangential to a perimeter of the opening in the distal portion and at least one other of the elongate slots can extend perpendicular to the perimeter of the opening in the distal portion. The device can have any number of variations. For example, the proximal portion and the plurality of flexible shields can be configured to rotate relative to the retractor when the proximal portion is coupled to the distal portion. For another example, a quantity of the plurality of flexible shields can be equal to or greater than a quantity of the plurality of access ports. For still another example, the proximal portion can be configured to rotate relative to the distal portion from an initial mating position, in which the proximal portion is freely releasable from the distal portion, to a second rotated position, in which the proximal portion is prevented from disengagement from the distal portion.

In another aspect, a method for providing access to a body cavity is provided that includes positioning a retractor in tissue such that the retractor forms a pathway through the tissue and into a body cavity, aligning a housing in a predetermined rotational orientation relative to the retractor to align a plurality of differing mating elements on the housing with a plurality of corresponding mating features on the retractor, and advancing the plurality of differing mating elements into the plurality of corresponding mating features to mate the housing to the retractor. The housing has a plurality of access ports configured to receive an instrument therethrough.

In some embodiments, the method can include rotating the housing relative to the retractor from an initial mating position, in which the plurality of differing mating elements are advanced into the plurality of corresponding mating features, to a second rotated position, in which the housing is mated to the retractor and the differing mating elements are out of alignment with the plurality of corresponding mating features on the retractor. The housing can be rotated relative to the retractor from the second rotated position until an alignment mechanism formed on at least one of the housing and the retractor indicates that the housing is in the initial mating position. The alignment mechanism can be in the initial mating position when a first protrusion extending laterally outward from a sidewall of the housing abuts a second protrusion extending laterally outward from a sidewall of the retractor.

The method can have any number of variations. For example, aligning the housing in a predetermined rotational orientation can include aligning an alignment feature on the housing with an alignment feature on the retractor with the alignment features aligning the plurality of mating elements on the housing with the plurality of corresponding mating features on the retractor. For another example, advancing the plurality of differing mating elements into the plurality of corresponding mating features can include advancing a plurality of feet extending distally from the housing into a plurality of proximally-facing openings formed in the retractor. The plurality of feet can each have a different size corresponding to a differently sized one of the proximally-facing openings. For yet another example, each sealing port can include an opening formed through the housing and have at least one sealing element disposed therein. Advancing the plurality of differing mating elements into the plurality of corresponding mating features can position a distal linear opening of at least one of the sealing elements tangential to a perimeter of an opening extending through the retractor and position a distal linear opening of at least one other of the sealing elements perpendicular to the perimeter of the opening extending through the retractor.

As another variation, in some embodiments the method can include advancing a plurality of flexible shields extending distally from the housing into a working channel extending through the retractor when the plurality of differing mating elements are advanced into the plurality of corresponding mating features. A plane of each of the flexible shields can extend tangential to a sidewall of the working channel. A quantity of the plurality of flexible shields can be equal to or greater than a quantity of the plurality of access ports.

In another embodiment, a method for providing access to a body cavity is provided that includes implanting a retractor having a working channel extending therethrough in tissue such that the retractor forms a pathway through the tissue and into a body cavity, and removably mating a housing having a plurality of sealing ports to the retractor such that a plurality of flexible shields each positioned adjacent to one of the sealing ports extend from the housing and into the working channel of the retractor. The method can vary in any number of ways. For example, the method can include rotating the housing and the flexible shields relative to the retractor when the housing is mated to the retractor. Rotating the housing and the flexible shields relative to the retractor can non-releasably mate the housing to the retractor. For another example, a plane of at least one of the flexible shields can extend tangential to a sidewall of the working channel and a plane of at least one other of the flexible shields can extend perpendicular to the sidewall of the working channel when the housing is removably mated to the retractor.
For yet another example, removably mating the housing to the retractor can include aligning the housing in a predetermined rotational orientation relative to the retractor. For still another example, each of the sealing ports can have a sealing element disposed therein that is configured to form a seal when no instrument is inserted therethrough. Each of the sealing elements can have an elongate slot formed at a distal end thereof, and a plane of each of the flexible shields can be parallel to at least one of the elongate slots.

In still another embodiment, a method for providing access to a body cavity is provided that includes positioning an alignment mechanism formed on a retractor positioned in tissue to form a pathway therethrough adjacent to an alignment mechanism formed on a housing having a plurality of sealing ports to position the housing in a predetermined rotational position relative to the retractor, engaging at least one mating mechanism formed on at least one of the housing and the retractor to releasably mate the housing and the retractor, and rotating the housing relative to the retractor to move the alignment mechanism formed on the housing a distance apart from the alignment mechanism formed on the retractor to thereby lock the housing to the retractor. The at least one mating mechanism can be engaged when the housing is locked to the retractor. In some embodiments, engaging the at least one mating mechanism can include advancing a plurality of differing mating elements on the housing into a plurality of corresponding mating features on the retractor.

The following is a list of embodiments of the present invention, which may or may not be claimed in the appended claim set:

Embodiment 1: A surgical access device, comprising: a retractor having a working channel extending therethrough for forming a pathway through tissue into a body cavity; and a housing having a plurality of sealing ports configured to receive an instrument therethrough and into the working channel, the housing being configured to releasably mate to the retractor in only one predetermined rotational orientation.

Embodiment 2: The device of embodiment 1, wherein the housing is configured to rotate relative to the retractor from an initial mating position, in which the housing is freely releasable from the retractor, to a second rotated position, in which the housing is prevented from disengagement from the retractor.

Embodiment 3: The device of embodiment 1, further comprising an alignment mechanism formed on at least one of the housing and the retractor and configured to indicate alignment of the housing and the retractor in the predetermined rotational orientation.

Embodiment 4: The device of embodiment 3, wherein the alignment mechanism comprises a first protrusion extending laterally outward from a sidewall of the housing and configured to align with a second protrusion extending laterally outward from a sidewall of the retractor.

Embodiment 5: The device of embodiment 1, wherein the housing has a plurality of mating elements that are configured to releasably engage the retractor to mate the housing to the retractor, and wherein at least one of the plurality of mating elements differs from another one of the plurality of mating elements.

Embodiment 6: The device of embodiment 5, wherein the mating elements rotatably mate the housing to the retractor.

Embodiment 7: The device of embodiment 5, wherein the plurality of mating elements comprise a plurality of feet extending distally from the housing and configured to engage a plurality of proximally-facing openings formed in the retractor.

Embodiment 8: The device of embodiment 7, wherein a proximal portion of the retractor has the plurality of proximally-facing openings formed therein, and wherein the plurality of feet are configured to engage the proximal portion of the retractor when the housing is rotated relative to the retractor from the predetermined rotational orientation.

Embodiment 9: The device of embodiment 7, wherein at least one of the plurality of proximally-facing openings formed in the retractor has a longitudinal length that differs from at least one other of the plurality of proximally-facing openings.

Embodiment 10: The device of embodiment 7, wherein each of the plurality of proximally-facing openings formed in the retractor has a size that differs from one another, and each of the plurality of feet has a size corresponding to one of the plurality of openings.

Embodiment 11: The device of embodiment 1, further comprising a flexible shield disposed within the retractor when the housing is mated to the retractor, the flexible shield being configured to protect the retractor from damage caused by insertion of a surgical instrument through the retractor.

Embodiment 12: The device of embodiment 11, wherein the flexible shield comprises a plurality of flexible strips extending distally from the housing.

Embodiment 13: The device of embodiment 1, wherein each sealing port comprises an opening formed through the housing and having at least one sealing element disposed therein, the at least one sealing element being configured to form at least one of a seal around an instrument inserted therethrough and a channel seal configured to form a seal when no instrument is inserted therethrough.

Embodiment 14: The device of embodiment 1, further comprising a plurality of housings, each housing having a plurality of sealing ports formed therein, and each housing being interchangeably, releasably matable to the retractor.

Embodiment 15: A surgical access device, comprising: a retractor having an opening extending therethrough for forming a pathway through tissue into a body cavity; and a housing rotatably coupled to the retractor and having a plurality of sealing ports in communication with the opening in the retractor, each of the sealing ports having a sealing element fixedly disposed therein, at least one of the sealing elements having a linear distal opening extending tangential to a perimeter of the opening in the retractor and at least one other of the sealing elements having a linear distal opening extending perpendicular to the perimeter of the opening in the retractor.

Embodiment 16: The device of embodiment 15, wherein two of the sealing elements have linear distal openings tangent to the perimeter of the opening.

Embodiment 17: The device of embodiment 15, wherein the housing is configured to be releasably mated to the retractor.

Embodiment 18: The device of embodiment 17, wherein the housing has a plurality of mating elements that are configured to releasably engage the retractor to mate the housing to the retractor, and wherein at least one of the plurality of mating elements differs from another one of the plurality of mating elements.

Embodiment 19: The device of embodiment 18, further comprising an alignment mechanism formed on at least one of the housing and the retractor and configured to indicate a single rotational orientation of the housing relative to the retractor in which the mating elements can releasably engage the retractor.

Embodiment 20: The device of embodiment 15, further comprising a plurality of flexible shields extending from the housing and configured to distally extend into the opening of the retractor when the housing is coupled to the retractor.

Embodiment 21: The device of embodiment 20, wherein the linear distal opening extending tangential to the perimeter of the opening in the retractor and the linear distal opening extending perpendicular to the perimeter of the opening in the retractor each extend parallel to a planar surface of at least one of the flexible shields.

Embodiment 22: A surgical access device, comprising: a proximal portion having a plurality of access ports formed therein for receiving surgical instruments therethrough; a distal portion removably coupled to the proximal portion and having an opening extending therethrough for forming a pathway through tissue into a body cavity; and a plurality of flexible shields extending from the proximal portion and configured to extend into the opening of the distal portion, each of the flexible shields being located adjacent to one of the access ports.

Embodiment 23: The device of embodiment 22, wherein the proximal portion and the plurality of flexible shields are configured to rotate relative to the retractor when the proximal portion is coupled to the distal portion.

Embodiment 24: The device of embodiment 22, wherein a quantity of the plurality of flexible shields is equal to or greater than a quantity of the plurality of access ports.

Embodiment 25: The device of embodiment 22, wherein each of the access ports has a sealing element disposed therein that is configured to form a seal when no instrument is inserted therethrough, each of the sealing elements having an elongate slot formed at a distal end thereof, and wherein a plane of each of the flexible shields is parallel to at least one of the elongate slots.

Embodiment 26: The device of embodiment 25, wherein each sealing element is in a substantially fixed position relative to the proximal portion, and when the proximal portion is coupled to the distal portion at least one of the elongate slots extends tangential to a perimeter of the opening in the distal portion and at least one of the elongate slots extends perpendicular to a perimeter of the opening in the distal portion.

Embodiment 27: The device of embodiment 25, wherein at least one of the elongate slots extends tangential to a perimeter of the opening in the distal portion and at least one other of the elongate slots extends perpendicular to the perimeter of the opening in the distal portion.

Embodiment 28: The device of embodiment 22, wherein the proximal portion is configured to rotate relative to the distal portion from an initial mating position, in which the proximal portion is freely releasable from the distal portion, to a second rotated position, in which the proximal portion is prevented from disengagement from the distal portion.

### BRIEF DESCRIPTION OF THE DRAWINGS

The invention will be more fully understood from the following detailed description taken in conjunction with the accompanying drawings, in which:

FIG. 1 is a perspective view of one embodiment of a surgical access device having sealing ports extending therethrough and having an alignment mechanism;

FIG. 2A is a perspective view of the device of FIG. 1 without an insufflation tube;

FIG. 2B is a perspective view of the device of FIG. 2A with a housing of the device rotated relative to a retractor of the device;

FIG. 3 is a bottom view of the device of FIG. 2A;

FIG. 4 is an exploded side view of the device of FIG. 2A;

FIG. 5 is an exploded perspective view of the device of FIG. 2A;

FIG. 6 is a perspective bottom view of a proximal housing of the device of FIG. 1;

FIG. 7 is a top view of the proximal housing of FIG. 6;

FIG. 8 is a perspective view of a proximal retractor base of the device of FIG. 1;

FIG. 9 is a perspective view of a housing of the device of FIG. 1 releasably mated to the proximal retractor base;

FIG. 10 is a bottom view of the housing and the proximal retractor base of FIG. 9;

FIG. 11 is a top view of the housing and the proximal retractor base of FIG. 9;

FIG. 12 is a side view of the housing and the proximal retractor base of FIG. 9;

FIG. 13 is a perspective view of a distal housing of the device of FIG. 1;

FIG. 14 is a bottom view of the distal housing of FIG. 13;

FIG. 15 is a partial perspective view of another embodiment of a surgical access device having a tabbed alignment mechanism;

FIG. 16 is a perspective view of another embodiment of a proximal retractor base;

FIG. 17 is a bottom view of the proximal retractor base of FIG. 16;

FIG. 18 is an exploded view of a sealing port of the device of FIG. 1;

FIG. 19 is an exploded view of another sealing port of the device of FIG. 1;

FIG. 20 is a bottom view of the housing of the device of FIG. 1;

FIG. 21 is a perspective view of another housing of a surgical access device having another arrangement of access ports extending therethrough;

FIG. 22 is a side view of another embodiment of a surgical access device having a plurality of safety shields;

FIG. 23 is a bottom view of a housing of the device of FIG. 22; and

FIG. 24 is a perspective view of another embodiment of a housing of a surgical access device having a plurality of safety shields.

### DETAILED DESCRIPTION OF THE INVENTION

Certain exemplary embodiments will now be described to provide an overall understanding of the principles of the structure, function, manufacture, and use of the devices and methods disclosed herein. One or more examples of these embodiments are illustrated in the accompanying drawings. Those skilled in the art will understand that the devices and methods specifically described herein and illustrated in the accompanying drawings are non-limiting exemplary embodiments and that the scope of the present invention is defined solely by the claims. The features illustrated or described in connection with one exemplary embodiment may be combined with the features of other embodiments. Such modifications and variations are intended to be included within the scope of the present invention.

Various exemplary methods and devices are provided for providing surgical access into a body cavity. In general, the methods and devices allow multiple surgical instruments to be inserted through independent access ports in a single surgical access device and into a body cavity. The instruments can be collectively rotatable about a central axis of the device, thus allowing for ease of manipulation within a patient's body. In one embodiment, a surgical access device includes a housing having multiple access ports or sealing ports for receiving surgical instruments, and a retractor removably coupled to the housing and having a working channel configured to extend into a body cavity. An alignment mechanism and/or mating features can be configured to align the housing and the retractor in a predetermined position relative to one another to allow for easy attachment and removal of the housing to and from the retractor. Once mated, the housing can rotate relative to the retractor, thereby helping to optimally position instruments inserted therethrough and into the body cavity in which the retractor extends. Each sealing port can include one or more sealing elements therein for sealing the port and/or forming a seal around a surgical instrument disposed therethrough. The sealing ports can be fixedly positioned relative to the housing to thereby position them relative to the working channel of the retractor regardless of the rotational position of the housing relative to the retractor. In some embodiments, the device can include a plurality of safety shields extending from the housing into the retractor to help protect the retractor from being damaged by instruments passed through the retractor's working channel.

As indicated above, the various surgical access devices can include a wound protector, cannula, ring retractor, or other member for forming a pathway through tissue (hereinafter generally referred to as a retractor). The retractor can extend from the housing and it can be configured to be positioned within an opening in a patient's body, such as the umbilicus. The sealing ports can each define working channels extending through the housing and aligned with the retractor. Any and all of the surgical access devices described herein can also include various other features, such as one or more ventilation ports to allow evacuation of smoke during procedures that utilize cautery, and/or one or more insufflation ports through which the surgeon can insufflate the abdomen to cause pneumoperitenium, as described by way of non-limiting example in U.S. Patent Application No. 2006/0247673 entitled "Multi-port Laparoscopic Access Device" filed November 2, 2006, which is hereby incorporated by reference in its entirety. The insufflation port can be located anywhere on the device, can have any size, and can accept a leur lock or a needle, as will be appreciated by those skilled in the art.

As discussed further below, any and all embodiments of a surgical access device can also include one or more safety shields positioned through, in, and around any of the components and/or tissue to protect the components against puncture or tear by surgical instruments being inserted through the device. In addition, any and all embodiments of a surgical access device can include engagement and release mechanisms that allow certain components of the surgical access device to be removable as needed.

In use, and as also further discussed below, the surgical access devices disclosed herein can provide access to a patient's body cavity. The retractor can be positionable within an opening in a patient's body such that a distal portion of the retractor extends into a patient's body cavity and a proximal portion configured to couple to the housing is positioned adjacent to the patient's skin on an exterior of the patient's body. A lumen in the retractor can form a pathway through the opening in a patient's body so that surgical instruments can be inserted from outside the body to an interior body cavity. The elasticity of the skin of the patient can assist in the retention of the retractor in the body opening or incision made in the body. The retractor can be placed in any opening within a patient's body, whether a natural orifice or an opening made by an incision. In one embodiment, the retractor can be substantially flexible so that it can easily be maneuvered into and within tissue as needed. In other embodiments, the retractor can be substantially rigid or substantially semi-rigid. The retractor can be formed of any suitable material known in the art, e.g., silicone, urethane, thermoplastic elastomer, and rubber.

Typically, during surgical procedures in a body cavity, such as the abdomen, insufflation is provided through the surgical access device to expand the body cavity to facilitate the surgical procedure. Thus, in order to maintain insufflation within the body cavity, most surgical access devices include at least one seal disposed therein to prevent air and/or gas from escaping when surgical instruments are inserted therethrough. Various sealing elements are known in the art, but typically the surgical access device can include at least one instrument seal that forms a seal around an instrument disposed therethrough, but otherwise does not form a seal when no instrument is disposed therethrough; at least one channel seal or zero-closure seal that seals the working channel created by the sealing port when no instrument is disposed therethrough; or a combination instrument seal and channel seal that is effective to both form a seal around an instrument disposed therethrough and to form a seal in the working channel when no instrument is disposed therethrough. A person skilled in the art will appreciate that various seals known in the art can be used including, e.g., duckbill seals, cone seals, flapper valves, gel seals, diaphragm seals, lip seals, iris seals, etc. A person skilled in the art will also appreciate that any combination of seals can be included in any of the embodiments described herein, whether or not the seal combinations are specifically discussed in the corresponding description of a particular embodiment.

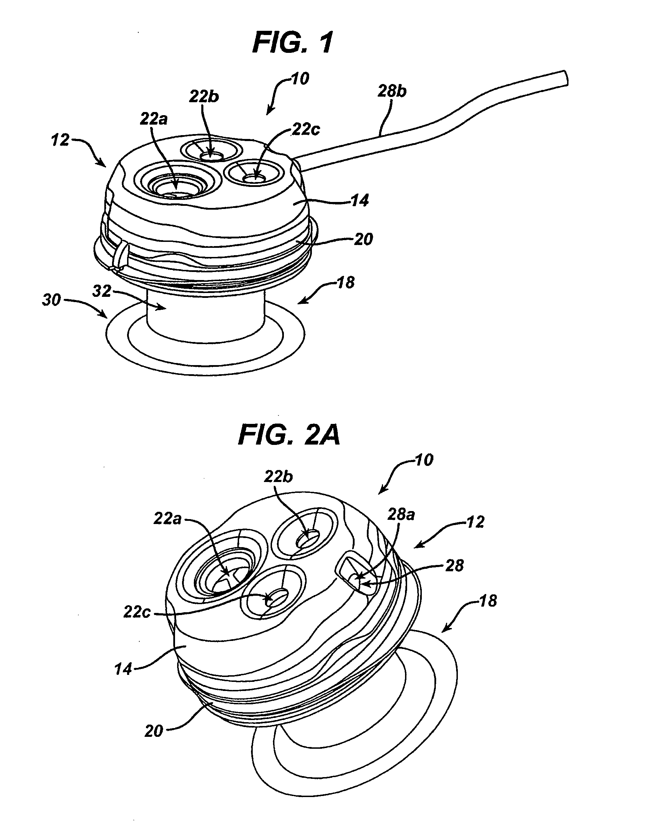

In an exemplary embodiment, shown in FIGS. 1-5, a surgical access device 10 is provided having a housing 12 configured to have one or more surgical instruments inserted therethrough. In this illustrated embodiment, the housing 12 includes a proximal housing 14, a seal base 16 that supports at least one sealing or access port, and a distal housing 20. While any number of sealing ports can be formed in the seal base 16, in this illustrated embodiment, first, second, and third sealing ports 22a, 22b, 22c extend through the seal base 16. The sealing ports 22a, 22b, 22c in this illustrated embodiment each have a central axis that extends substantially perpendicular to a plane of the seal base 16, and the sealing ports 22a, 22b, 22c are each in a fixed position relative to the housing 12, but any one or more of the sealing ports can be angled relative to the seal base 16 and/or rotatable or otherwise movable relative to the seal base 16 and/or other portion(s) of the housing 12. The housing 12 can be removably coupled to a retractor 18 configured to distally extend from the housing 12 and to provide a pathway through tissue into a body cavity. In this embodiment, the retractor 18 includes a proximal retractor portion or proximal retractor base 24 coupled to a distal retractor portion 26. The housing 12 can be movable with respect to the retractor 18, as will be discussed in more detail below. Such a configuration can help facilitate instrument positioning in a body cavity to which the device 10 provides access.

The device 10 can also include an insufflation port 28 supported by the proximal housing 14, although a person skilled in the art will appreciate that the insufflation port 28 can be located elsewhere in the housing 12 or in other locations. A person skilled in the art will also appreciate that the insufflation port 28 can have a variety of configurations. Generally, the insufflation port 28 can be configured to pass an insufflation fluid through an insufflation orifice 28a of the insufflation port 28 through a flexible insufflation tube 28b (only shown in FIG. 1) into and/or out of a body cavity to which the device 10 provides access.

The housing 12 of the surgical access device 10 can have a variety of configurations. As shown in this embodiment, the proximal housing 14 is in the form of a seal cap configured to releasably mate the seal base 16 to the retractor 18, and the distal housing 20 is in the form of an o-ring configured to be disposed between the seal base 16 and the retractor 18 to form a seat and seal between the base 16 and a distal portion of the device 10, e.g., the retractor 18. The retractor 18, the seal base 16, the proximal housing 14, and the distal housing 20 can each have various sizes, shapes, and configurations, as discussed further below.

As noted above, the retractor 18 can extend distally from the housing 12, and it can be configured to be positioned in an opening formed in tissue. The retractor 18 can, as shown in this exemplary embodiment, include a substantially flexible distal portion 26 having a proximal flange 29 and a distal flange 30 with an inner elongate portion 32 extending therebetween. The inner elongate portion 32 can have a diameter less than a diameter of the proximal and distal flanges 29, 30, which can have the same diameter or different diameters from one another. The proximal flange 29 can be configured to be seated within the proximal retractor base 24 and optionally attached thereto using an adhesive, sealant, complementary threads, or any other attachment mechanism, as will be appreciated by a person skilled in the art. A proximal o-ring can be optionally positioned within the proximal flange 29 to help provide structural support to the retractor 18 within the proximal retractor base 24. A distal o-ring can optionally be positioned within the distal flange 30 to provide structural support to the retractor 18 within a patient's body. The proximal and distal o-rings can be substantially flexible or substantially rigid as needed, same or different from one another, for use in a particular application.

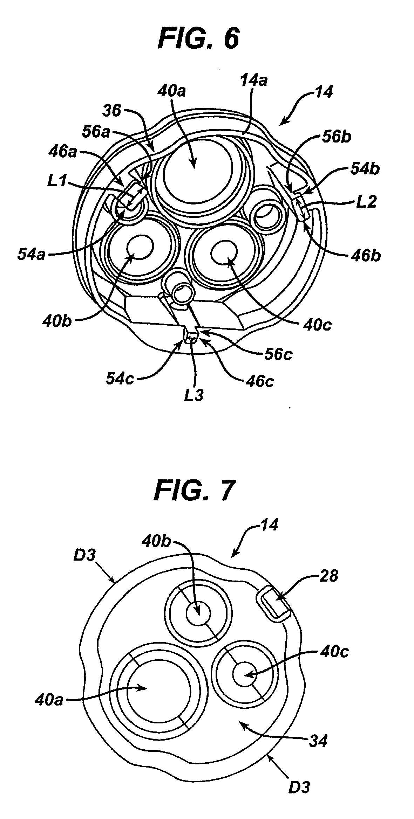

The seal cap 14 can, as illustrated in FIGS. 6 and 7, have a proximal surface 34 and a circumferential wall 36 extending distally from the proximal surface 34. The circumferential wall 36 can optionally include one or more cut-out portions (not shown) formed therein adjacent to a sealing port that are configured to help angle surgical instruments inserted through the sealing ports 22a, 22b, 22c.

In any and all of the surgical access device embodiments disclosed herein, an engagement and/or release mechanism can be included to allow a seal base to be separated from a seal cap, to allow a housing to be separated from a retractor, and/or to allow a seal port to be separate from a seal base. Any engagement and release mechanism known in the art, e.g., a snap-lock mechanism, corresponding threads, etc., can be used to releasably mate two components of the device 10. In one embodiment, the engagement and release mechanism can include a latch mechanism, as described by way of non-limiting example in U.S. Application No. 12/242,765 entitled "Surgical Access Device" filed on September 30, 2008, which is hereby incorporated by reference in its entirety.

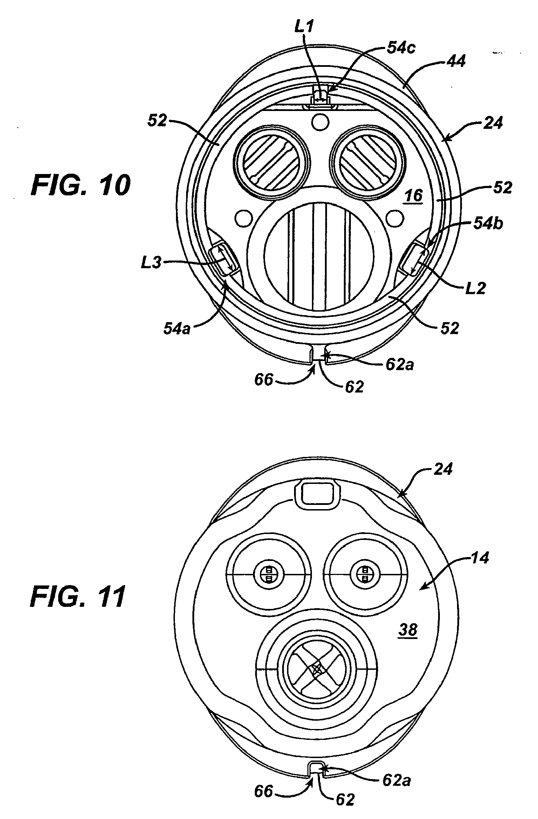

As illustrated in the embodiment shown in FIGS.1-12, the housing 12 and the retractor 18 can include an engagement and release mechanism in the form of a bayonet latch mechanism.
At least one mating feature in the form of a bayonet foot or pin, e.g., three radially arranged bayonet feet or pins 46a, 46b, 46c spaced equidistantly or any other distance apart, as shown in FIG. 6, can distally extend any length from an inner circumference or other portion of the housing 12, e.g., from an inner surface of the circumferential wall 36 of the cap 14. The bayonet feet 46a, 46b, 46c can have any shape and size. The bayonet feet 46a, 46b, 46c can be the same as each other, but in an exemplary embodiment, at least one of the bayonet feet 46a, 46b, 46c can differ from at least one other of the bayonet feet 46a, 46b, 46c, e.g., have a differing shape, have a differing size, extend a different distal distance from the cap 14, etc. In this illustrated embodiment, each of the bayonet feet 46a, 46b, 46c are L-shaped with arms 56a, 56b, 56c distally extending from the cap 14 and ledges 54a, 54b, 54c extending substantially perpendicular and radially outward from the arms 56a, 56b, 56c. The arms 56a, 56b, 56c can also extend radially inward from an inner surface of the cap 14, as illustrated. Each of the bayonet feet 46a, 46b, 46c in this embodiment, as shown in FIGS. 6 and 10, have a different size with their ledges 54a, 54b, 54c having respective first, second, and third longitudinal lengths L1, L2, L3. The first longitudinal length L1 is greater than the second longitudinal length L2 and the second longitudinal length L2 is greater than the third longitudinal length L3, although as mentioned above, any of the ledges 54a, 54b, 54c can have the same size as any of the other ledges 54a, 54b, 54c.

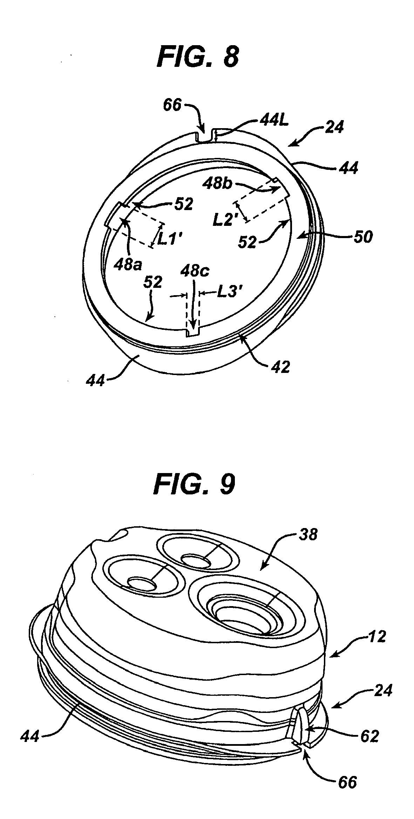

The bayonet feet 46a, 46b, 46c can be configured to engage corresponding mating features in the form of one or more cut-outs, slots, or openings 48a, 48b, 48c formed in a ring member 50 of the proximal retractor base 24. The ring member 50 can form a proximal surface of the proximal retractor base 24, as shown in FIG. 8, such that the openings 48a, 48b, 48c are formed in a proximal surface of the retractor 18, or the ring member 50 can be formed elsewhere, e.g., as an inner ring formed in a proximal or distal portion of the proximal retractor base 24. Similar to that discussed above regarding the bayonet feet 46a, 46b, 46c, the openings 48a, 48b, 48c can have any shape and size and can be the same or different as any one or more other ones of the openings 48a, 48b, 48c. In the illustrated embodiment, the openings 48a, 48b, 48c each have a different longitudinal length L1', L2', L3' respectively corresponding to the longitudinal lengths L1, L2, L3 of the bayonet feet 46a, 46b, 46c. A person skilled in the art will appreciate that the longitudinal lengths L1', L2', L3' of the openings 48a, 48b, 48c can be slightly larger than the corresponding longitudinal lengths L1, L2, L3 of the bayonet feet 46a, 46b, 46c to allow the bayonet feet 46a, 46b, 46c to respectively receive the bayonet feet 46a, 46b, 46c therein.

The bayonet feet 46a, 46b, 46c can be configured to be lowered into the corresponding openings 48a, 48b, 48c in the proximal retractor base 24, as illustrated in FIG. 10. If any one or more of the bayonet feet 46a, 46b, 46c differ from one another and one or more of the openings 48a, 48b, 48c correspondingly differ, the housing 12 can be configured to mate to the retractor 18 in one or more predetermined rotational orientations, e.g., with different circumferentially arranged bayonet feet 46a, 46b, 46c aligned with their corresponding different circumferentially arranged openings 48a, 48b, 48c. In the illustrated embodiment where each of the bayonet feet 46a, 46b, 46c differs from one another and each of the openings 48a, 48b, 48c correspondingly differs from one another, the cap 14 can only be positioned in one predetermined rotational orientation relative to the proximal retractor base 24 where the bayonet feet 46a, 46b, 46c can each be simultaneously lowered into the corresponding openings 48a, 48b, 48c. With the bayonet feet 46a, 46b, 46c engaging the openings 48a, 48b, 48c, the housing 12 can be releasably mated to the retractor 18, e.g., the cap 14 can be attached to or removed from the proximal retractor base 24.

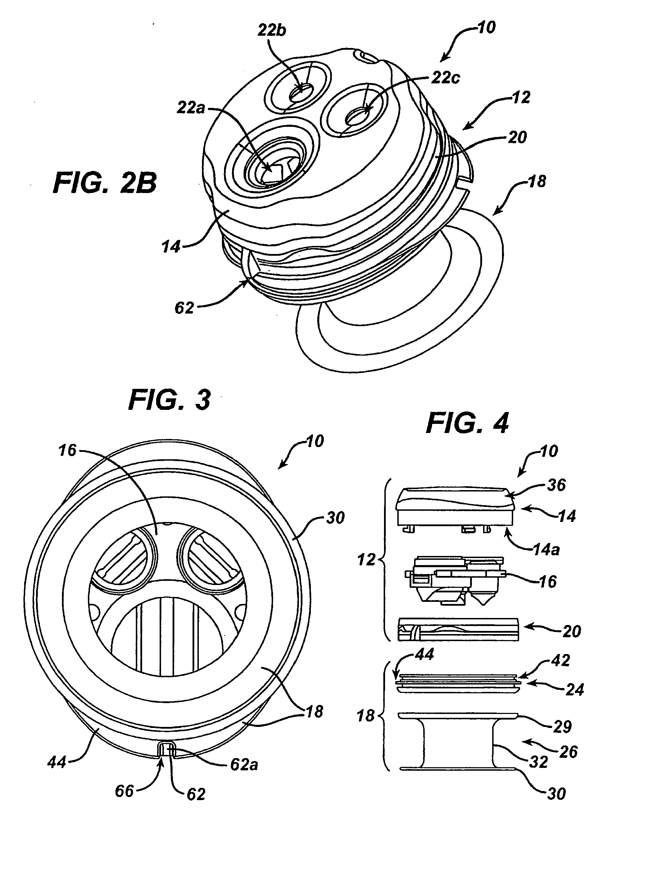

With the bayonet feet 46a, 46b, 46c engaging the corresponding openings 48a, 48b, 48c, e.g., with the device 10 in a default or initial mating position shown in FIG. 2A, the housing 12can be rotated in a first direction, e.g., a counterclockwise direction, relative to the retractor 18, thereby causing the bayonet feet 46a, 46b, 46c to travel laterally within the openings 48a, 48b, 48c to a position in which ledges 52 of the proximal retractor base 24 that extend between the openings 48a, 48b, 48c cover corresponding ledges 54a, 54b, 54c on the bayonet feet 46a, 46b, 46c, thereby securing or locking the seal cap 14 to the proximal retractor base 24 in a rotated, non-releasable position, e.g., with the device 10 in a rotated position shown in FIG. 2B. The housing 12, e.g., the cap 14, can optionally include surface features, e.g., ridges, bumps, textured surface, etc., to help facilitate gripping and turning of the housing 12.

With the cap 14 non-releasably attached, i.e., rotated from the initial mating position, to the proximal retractor base 24, the housing 12 can be rotated in the first direction and/or in a second opposite direction, e.g., a clockwise direction, to rotate the housing 12 relative to the retractor 18. While the housing 12 can be configured to be rotatable relative to the retractor 18 in only one of the first and second directions, the housing 12 as illustrated is rotatable in both the first and second directions, which can help more effectively position surgical instruments inserted through the housing 12 with respect to each other. The housing 12 can be configured to rotate 360° relative to the retractor 18, although the device 10 can include a mechanism that can limit the housing's rotation relative to the retractor 18 to less than 360°, as discussed further below. Having one or more different bayonet feet 46a, 46b, 46c and corresponding openings 48a, 48b, 48c, the device 10 can be configured to allow rotational motion of the housing 12 relative to the retractor 18 without the cap 14 detaching from the retractor 18 unless the housing 12 is in a predetermined rotational orientation relative to the retractor 18 that allows the housing 12 to releasably mate thereto, e.g., unless the differently sized bayonet feet 46a, 46b, 46c are aligned with same sized openings 48a, 48b, 48c.

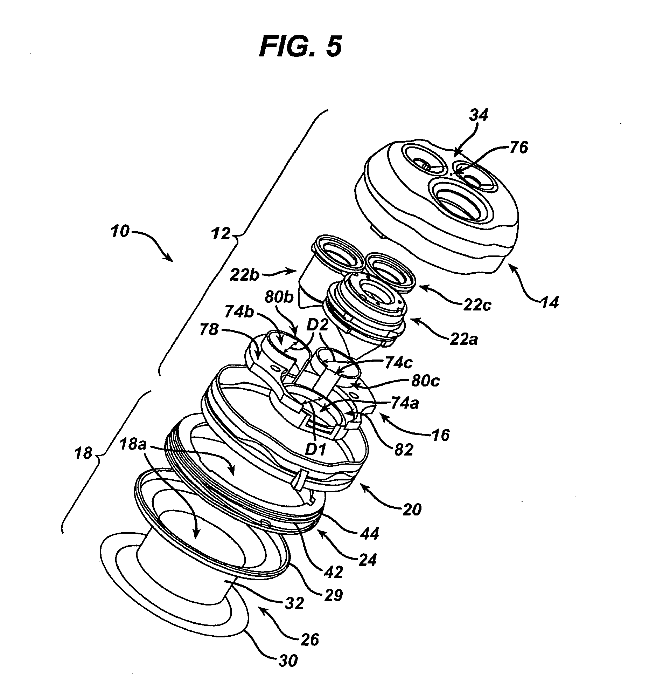

If disengagement of the housing 12 and the retractor 18 is desired, e.g., to replace the seal base 16 with another seal base having a different number or different sizes of sealing ports, to withdraw waste material from the body of the patient through a working channel 18a of the retractor 18 (shown in FIG. 5), or to replace or remove the retractor 18, the housing 12 can be rotated in the first and/or second directions to engage the bayonet feet 46a, 46b, 46c with the corresponding openings 48a, 48b, 48c. With the bayonet feet 46a, 46b, 46c engaging the corresponding openings 48a, 48b, 48c, the cap 14 is in the one predetermined rotational orientation relative to the proximal retractor base 24 such that the bayonet feet 46a, 46b, 46c can be withdrawn from the corresponding openings 48a, 48b, 48c to release the housing 12 from the retractor 18.

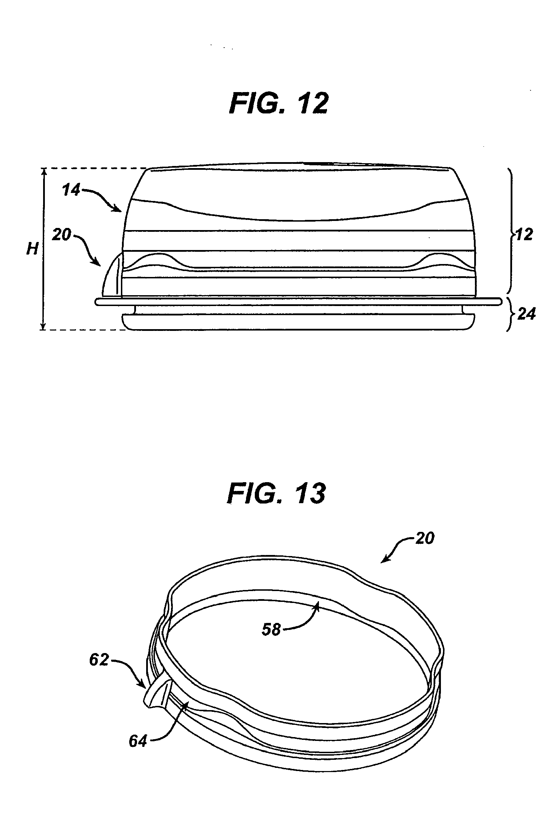

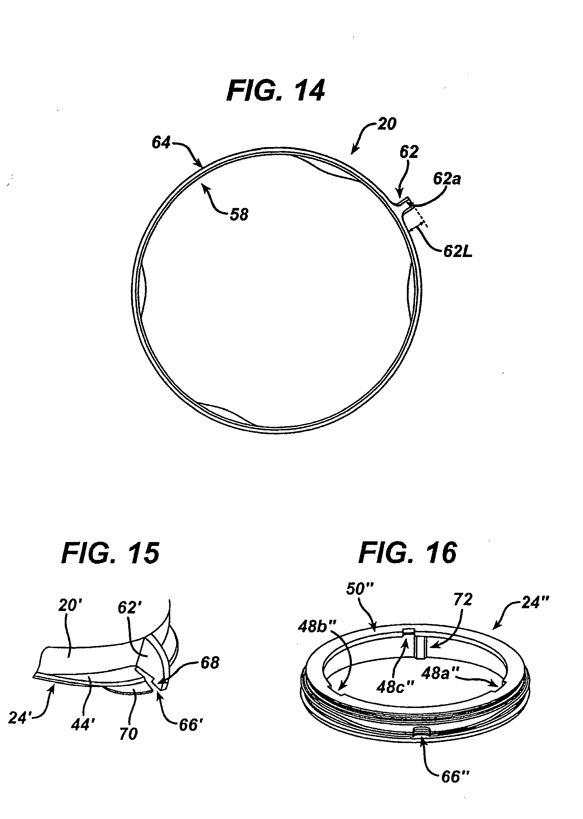

As indicated above, the distal housing 20 can be positioned between the seal base 26 and the retractor 18. Although the distal housing 20 can have a variety of sizes, shapes, and configurations, the distal housing 20 can, as shown in FIGS. 13 and 14, be in the form of a substantially circular o-ring. The distal housing 20 can be coupled between a distal surface 14a of the cap 14 (shown in FIGS. 4 and 6) and a complementary lip 44 formed on and extending radially outward from the proximal retractor base 24. The complementary lip 44 can continuously run circumferentially around the proximal retractor base 24 as shown, or the complementary lip 44 can run around one or more discrete portions of the proximal retractor base 24. The distal housing 20 can be removably or fixedly attached to the cap 14, or the distal housing 20 and the cap 14 can be integrally formed as a single member. In this illustrated embodiment, the cap 14 and the distal housing 20 are fixedly attached together using an attachment mechanism such as an adhesive. At least a distal portion of a circumferential interior wall 58 of the distal housing 20, shown in FIGS. 13 and 14, can have a size and shape corresponding to a proximal circumferential wall 42 of the proximal retractor base 24, shown in FIG. 8, such that at least a portion of the circumferential interior wall 58 can be configured to engage the proximal circumferential wall 42. A distal surface 60 of the distal housing 20 can have a size and shape corresponding to the complementary lip 44 such that the distal surface 60 of the distal housing 24 can be configured to engage the lip 44. The distal housing 20 with the distal surface 60 engaging the lip 44 and the circumferential interior wall 58 engaging the circumferential wall 42 can thereby help stabilize coupling of the housing 12 to the retractor 18 when the cap 14 is attached to the proximal retractor base 24, as illustrated in FIGS. 9-12, and can help provide a seat and seal between the seal base 16 and the retractor 18.

As mentioned above, the distal housing 20 can include an alignment mechanism configured to indicate alignment of the housing 12 and the retractor 18. A person skilled in the art will appreciate that the alignment mechanism can be formed on any portion of the housing 12, e.g., the cap 14, and/or on any portion of the retractor 18, e.g., on the proximal retractor base 24. A person skilled in the art will also appreciate that the housing 12 and/or the retractor 18 can include one or more same or differing alignment mechanisms, e.g., spaced equidistantly or any other distance apart around a perimeter of the housing 12 and/or the retractor 18. The alignment mechanism can have a variety of configurations. In one embodiment, the alignment mechanism can include a feature formed on the distal housing 20 that can indicate alignment of the distal housing 20 with the retractor 18, e.g., in the predetermined rotational orientation to allow mating of the two components. The feature can include a visually identifiable element formed on the distal housing 20 such as a colored dot, line, or other shape, a groove or other depression, a hemispherical dimple or other protrusion, an alphabetical or numerical character, etc. While the alignment mechanism can be formed anywhere on the housing 12 as mentioned above, in an exemplary embodiment, the alignment mechanism can be located on an opposite side of the housing 12 than the insufflation port 28, e.g., about 180° away around a circumference of the housing 12, to help reduce interference therebetween.

In the illustrated embodiment, the alignment mechanism includes a feature in the form of a protrusion 62 extending laterally outward from a sidewall of the distal housing 20, e.g., from an outer circumferential wall 64 of the distal housing 20. The protrusion 62 can have a variety of sizes, shapes, and configurations. As shown in FIG. 13, the protrusion 62 has an arcuate cross-sectional shape as a sector of an ellipse, although the protrusion 62 can have any shape. The protrusion 62 can also have any size. In an exemplary embodiment the protrusion 62 can laterally extend a distance 62L (see FIG. 14) from the outer circumferential wall 64 of the distal housing 20. As shown in FIGS. 3, 9, and 10, the lateral distance 62L can be equal to or less than a lateral distance 44L that the lip 44 extends laterally outward from the proximal retractor base 24 such that the protrusion 62 does not extend laterally beyond the lip 44 when the housing 12 is attached to the retractor 18 at least when the bayonet feet 46a, 46b, 46c engage the corresponding openings 48a, 48b, 48c, e.g., when the housing 12 is in the default position and is releasably mated with the retractor 18 in a predetermined rotational orientation.

The protrusion 62 can be configured to align or to engage, e.g., abut, extend into, or otherwise contact, a corresponding alignment feature formed on the retractor 18. The alignment feature on the retractor 18 can also have a variety of sizes, shapes, and configurations, e.g., a colored dot, line, or other shape, a groove or other depression, a hemispherical dimple or other protrusion, an alphabetical or numerical character, etc., and it can be the same as or different from the alignment feature formed on the housing 12. In the illustrated embodiment, the alignment feature on the retractor 18 includes a cut-out 66 formed in the retractor 18. The cut-out 66 in this embodiment, as shown in FIGS. 8-11, is formed in the circumferential lip 44 of the proximal retractor base 24. The cut-out 66 can have any size and shape, but in the illustrated embodiment, the cut-out 66 can have a size and shape, e.g., substantially rectangular, corresponding to a shape of a distal surface 62a of the protrusion 62 formed on the distal housing 20.

When the alignment mechanism is aligned or engaged, the protrusion 62 on the distal housing 20 can align with the cut-out 66. Because the protrusion 62 and the cut-out 66 each have fixed positions relative to the housing 12 and the retractor 18, respectively, and because the bayonet feet 46a, 46b, 46c and the corresponding openings 48a, 48b, 48c also have fixed positions relative to the housing 12 and the retractor 18, respectively, engagement of the alignment feature can indicate a relative positioning of the bayonet feet 46a, 46b, 46c and the corresponding openings 48a, 48b, 48c. In other words, when the protrusion 62 is aligned with the cut-out 66, as shown in FIG. 2A, the bayonet feet 46a, 46b, 46c can engage the corresponding openings 48a, 48b, 48c such that the housing 12 can be releasably mated to the retractor 18, e.g., the housing 12 can be removed from or attached to the retractor 18. Correspondingly, when the housing 12 is mated to the retractor 18 and the alignment mechanism is not engaged, e.g., when the protrusion 62 and the cut-out 66 are out of alignment as shown in FIG. 2B, the bayonet feet 46a, 46b, 46c are also out of alignment from the corresponding openings 48a, 48b, 48c such that the housing 12 can be non-releasably, rotatably mated to the retractor 18, e.g., the housing 12 cannot be removed from or attached to the retractor 18 but can be rotated relative thereto. The housing 12 can be rotated relative to the retractor 18 to reengage the alignment mechanism to allow the housing 12 to be decoupled from the retractor 18.

A surgical access device can optionally include a stop mechanism configured to indicate with a greater degree of certainty engagement of the alignment mechanism. The stop mechanism can have a variety of sizes, shapes, and configurations. FIG. 15 illustrates one embodiment of an alignment mechanism and a stop mechanism. Similar to the alignment mechanism of FIGS. 9-11, this illustrated embodiment of an alignment mechanism includes a tabbed protrusion 62' extending laterally outward from a sidewall of a distal housing 20' that is configured to engage a corresponding opening 66' formed in a lip 44' formed on and extending radially outward from a proximal retractor base 24'. The tabbed protrusion 62' can include a tab 68 distally extending from an end of the protrusion 62' opposite from an end of the protrusion 62' that is attached to the distal housing 20'. The tab 68 is shown as a box-shaped protrusion distally extending from an arcuate protrusion similar to the protrusion 62 discussed above, but the tab 68 can have any shape. The tab 68 can be configured to engage an extension 70 extending radially outward from the lip 44' such that when the tab 68 engages the extension 70, the distal housing 20', and hence the housing (not shown) of which it is part, cannot be rotated in one direction, e.g., clockwise, relative to the retractor (not shown) of which the proximal retractor base 24' is part. The extension 70 is shown as an arcuate shape as a sector of an ellipse, but the extension 70 can have any shape. Because the stop mechanism can be configured to stop rotational motion of the housing relative to the retractor, the stop mechanism can thereby limit rotation of the housing relative to the retractor to less than 360°, e.g., to about 355°. The housing can nevertheless be configured to be mated to the retractor and rotate in multiple directions relative to the retractor, e.g., clockwise and counterclockwise, when the stop mechanism is not engaged. When the stop mechanism is engaged, e.g., when the tab 68 contacts or abuts the extension 70, the stop mechanism can provide confirmation that the alignment mechanism is engaged such that the housing can be released from the retractor. Thus, when the housing is rotated relative to the retractor such that the alignment mechanism is disengaged, the housing can be rotated relative to the retractor until the tab 68 abuts the extension 70, thereby indicating that the housing is in a predetermined rotational orientation that allows the housing to be removed from the retractor.

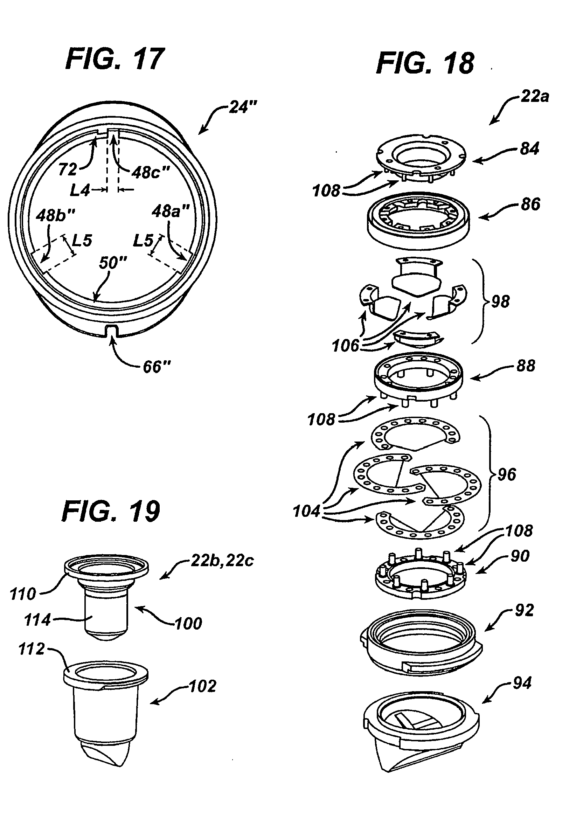

FIGS. 16 and 17 illustrate another embodiment of an alignment mechanism and a stop mechanism. As shown in this embodiment, a proximal retractor base 24" can be similar to the proximal retractor base 24 discussed above but can include a protrusion 72 extending laterally inward from an inner sidewall of the proximal retractor base 24" that is configured to engage a corresponding feature formed on a housing, e.g., the housing 12 of FIG. 5 that includes the cap 14 of FIG. 6 having the bayonet feet 46a, 46b, 46c, that is configured to be releasably mated to a retractor including the proximal retractor base 24". As in the illustrated embodiment, the protrusion 72 can be located adjacent to one of a plurality of mating features in the form of cut-outs, slots, or openings 48a", 48b", 48c" formed in a ring member 50" of the proximal retractor base 24", although the protrusion 72 can be located anywhere around the perimeter of the inner sidewall of the proximal retractor base 24". FIGS. 16 and 17 also illustrate an embodiment of a retractor having one opening 48c" with a first longitudinal length L4 that is less than a second longitudinal length L5 of each of the other openings 48a", 48b". The protrusion 72 is shown as a box-shaped member extending between proximal and distal ends of the proximal retractor base's inner sidewall, but the protrusion 72 can have any shape and size.

The protrusion 72 can be configured to allow the bayonet feet 46a, 46b, 46c to engage the openings 48a", 48b", 48c" as discussed above and to contact or abut one of the bayonet feet 46a, 46b, 46c to prevent rotation of the housing 12 relative to the retractor including the proximal retractor base 24" to indicate engagement of the alignment mechanism, e.g., the protrusion 62 of the housing 12 that is configured to engage a cut-out 66" formed in the proximal retractor base 24". The stop mechanism can thus be a separate member from the alignment mechanism and/or can be positioned at a location different from the alignment mechanism, e.g., about 180° away around a circumference of the proximal retractor base 24" as in this illustrated embodiment. When the housing 12 is releasably mated to the proximal retractor base 24", the protrusion 72 can be configured to prevent rotation of the housing 12 relative thereto in the first direction, e.g., clockwise, but to allow rotation relative thereto in the second direction, e.g., counterclockwise, thereby indicating engagement of the alignment mechanism. The housing 12 can be rotated relative to the retractor in the second direction to disengage the alignment mechanism such that the bayonet feet 46a, 46b, 46c can travel laterally within the openings 48a", 48b", 48c" to a position in which ledges 52" of the proximal retractor base 24" that extend between the openings 48a", 48b", 48c" cover the corresponding ledges 54a, 54b, 54c on the bayonet feet 46a, 46b, 46c, thereby securing or locking the seal cap 14 to the proximal retractor base 24" in a rotatable, non-releasable position. The housing 12 can then be rotated relative to the retractor in the first and/or second directions until one of the bayonet feet 46a, 46b, 46c contacts or abuts the protrusion 72 inside the retractor, thereby indicating reengagement of the alignment mechanism. The bayonet feet 46a, 46b, 46c can be configured such that only one of the bayonet feet 46a, 46b, 46c is configured to abut or contact the protrusion 72, which can help increase possible rotational motion of the housing 12 relative to the retractor, e.g., about 355° therearound. For non-limiting example, one of the ledges 54a, 54b, 54c of the bayonet feet 46a, 46b, 46c can extend radially outward a longer distance than the other ledges 54a, 54b, 54c of the bayonet feet 46a, 46b, 46c such that only that one longer ledge can engage the protrusion 72.

The seal base 16 can have a variety of sizes, shapes, and configurations, as can the sealing ports 22a, 22b, 22c seated therein. As shown in FIGS. 4 and 5, the seal base 16 can include one or more seal port cut-outs or openings 74a, 74b, 74c formed therein for respectively receiving the sealing ports 22a, 22b, 22c. The openings 74a, 74b, 74c can correspond to the openings 40a, 40b, 40c formed in the cap 14 such that surgical instruments can be inserted into the cap openings 40a, 40b, 40c and through the sealing ports 22a, 22b, 22c seated in the base 16. In some embodiments, the seal base 16 can also have an insufflation port cut-out or opening formed therethrough for seating an insufflation port if an insufflation port opening is formed in the proximal surface 34 of the cap 14. A person skilled in the art will appreciate that there can be any number of port openings formed in the seal base 16 that can be arranged in any way in the base 16. As shown in the illustrated embodiment, the cap openings 40a, 40b, 40c and the base port openings 74a, 74b, 74c, and hence also the sealing ports 22a, 22b, 22c, can be radially arranged around a central axis or center-point of the housing 12, e.g., a central axis or center-point 76 of the cap 14, such that each of the sealing ports 22a, 22b, 22c can have a central axis that differs from central axes of the other sealing ports 22a, 22b, 22c.

The port openings 74a, 74b, 74c can also have any combination of sizes and shapes. As shown, the port openings 74a, 74b, 74c can each have a shape corresponding to a shape of the sealing port 22a, 22b, 22c seated therein, which in this illustrated embodiment is substantially circular for each of the openings 74a, 74b, 74c. The first port opening 74a for seating the first sealing port 22a can have a first diameter D1 that is larger than a second diameter D2 of the second and third port openings 74b, 74c for respectively seating the second and third sealing ports 22b, 22c. For non-limiting example, the cap 14 can have a diameter D3 (see FIG. 7) of about 60 mm, the second diameter D2 can be about 5 mm, and the first diameter D1 can be about 15 mm, e.g., about three times the second diameter D2.

In some embodiments, a proximal base surface of the seal base can be substantially flat with port openings being formed in a same plane with each other, either co-planar parallel to the proximal base surface or recessed in the seal base. In other embodiments, such as the one illustrated in FIG. 5, a proximal base surface 78 of the seal base 16 can be non-planar with at least one recessed portion extending in a plane distally displaced from and parallel to a plane of the proximal base surface 78 and/or at least one raised portion proximally displaced from and parallel to a plane of the proximal base surface 78. The base 16 can also have port openings formed in the plane of the proximal base surface 78. The seal base's one or more recessed portions and one or more raised portions can help compensate for sealing elements of different lengths to help prevent distal seal element openings of each of the sealing elements from contacting an interior of the retractor 18, as discussed below, at least when the surgical access device 10 is in a default position, e.g., as illustrated in FIGS. 1, 2A and 3, and at least when the device 10 is not positioned in tissue and has no surgical instruments inserted therethrough.

In this illustrated embodiment, the seal base 16 has two raised or proximally extending housings 80b, 80c in which the second and third port openings 74b, 74c are formed. The raised housings 80b, 80c can have any height, same or different from any other raised housings, configured to help provide clearance room for the second and third sealing elements of the second and third sealing ports 22b, 22c seated in the second and third port openings 74b, 74c positioned above the proximal base surface 78 to help prevent the second and third sealing elements from contacting the retractor 18, as discussed below, at least when the surgical access device 10 is in the default position.

The illustrated seal base 16 also has one recessed portion 82 in which the first port opening 74a is formed. The recessed portion 82 can be recessed any depth below the proximal base surface 78, and it can be configured to allow a relatively small sealing element to extend through the base 16 and have its distal end substantially co-planar with distal ends of any other sealing elements extending through the base 16.

The sealing ports 22a, 22b, 22c can be attached or mated to the seal base 16 using any attachment or mating mechanism known in the art, but in the illustrated embodiment the sealing ports 22a, 22b, 22c can each mate with the seal base 16 through an interference fit between the base 16 and the cap 14. In general, the first sealing port 22a can include a port housing, which can be seated directly or indirectly in a port opening in the seal base 16, and a sealing element, which can be positioned within an associated port housing. A sealing element can include at least one instrument seal and/or at least one channel seal, and can generally be configured to contact an instrument inserted through the sealing element's associated sealing port. The second and third sealing ports 22b, 22c can each generally include a sealing element and a port housing configured to be positioned within an associated sealing element.

As shown in FIGS. 5, 18, and 19, the first sealing port 22a can include a first port housing, which can be seated within the first port opening 74a in the seal base 16, and a first sealing element, which can be positioned within the first port housing. The first port housing can include a crown 84, a washer 86, a gasket ring 88, a retainer ring 90, and a spacer seal 92. The first sealing element can include a first distal seal 94 and a proximal seal including a multi-layer conical seal 96 positioned proximal to the first distal seal 94 and a multi-layer protective member 98 disposed on a proximal surface of the conical seal 96. The second and third sealing ports 22b, 22c can each respectively include second and third port housings, which can be respectively seated within the second and third port openings 74b, 74c in the seal base 16, and second and third sealing elements, which can be respectively positioned within the second and third port housings. The second and third port housings can each include a deep cone 100. The second and third sealing elements can include a second distal seal 102. Although the second and third sealing ports 22b, 22c are configured and used similar to each other in this embodiment, a person skilled in the art will appreciate that the second and third sealing ports 22b, 22c can be configured different from one another.

The various port housings and sealing elements of the sealing ports 22a, 22b, 22c can have a variety of sizes, shapes, and configurations. A person skilled in the art will appreciate that while channel or zero-closure seals in the form of duckbill seals are shown for the first and second distal seals 94, 102, any seal, e.g., duckbill seals, cone seals, flapper valves, gel seals, diaphragm seals, lip seals, iris seals, non-linear sealing elements such sealing elements with an S-shaped opening, etc., same or different from any other of the other distal seals 94, 102 can be used and can be aligned in any way relative to the base 16. Generally, a zero-closure seal can be configured to form a seal in a working channel when no instrument is disposed therethrough to thus prevent the leakage of insufflation gases delivered through the surgical access device to the body cavity. A duckbill seal can generally have opposed flaps that extend at an angle toward one another in a distal direction and that come together at a distal end to form a seal face. The opposed flaps can be movable relative to one another to allow the seal face to move between a closed position, in which no instrument is disposed therethrough and the seal face seals the working channel of the surgical access device, and an open position in which an instrument is disposed therethrough. A duckbill seal can include various other features, as described in more detail in U.S. Application No. 11/771,263, entitled "Duckbill Seal with Fluid Drainage Feature," filed on June 29, 2007, which is hereby incorporated by reference in its entirety. In addition, the seal face of the duckbill seal can be in any nonlinear shape or configuration known in the art, for example in an S-shaped configuration, as described in more detail in U.S. Patent No. 5,330,437, entitled "Self Sealing Flexible Elastomeric Valve and Trocar Assembly for Incorporating Same," filed November 12, 1993, which is hereby incorporated by reference in its entirety.

As mentioned above and as illustrated in FIG. 18, the first sealing port 22a can include the first port housing and the first sealing element. The multi-layer conical seal 96 of the first sealing element can include a series of overlapping seal segments 104 that are assembled in a woven arrangement to provide a complete seal body. The seal segments 104 can be stacked on top of one another or woven together in an overlapping fashion to form the multi-layer seal 96 having a central opening (not shown) therein. The seal segments 104 can be made from any number of materials known to those skilled in the art, but in an exemplary embodiment the seal segments 104 are formed from an elastomeric material. The multi-layer protective member 98 can similarly be formed from a series of overlapping segments 106 that are disposed proximal to the overlapping seal segments 104 and that are configured as anti-eversion elements to protect the seal segments 104 from damage caused by surgical instruments passed through the opening in the multi-layer seal 96. The protective member 98 can also be formed from various materials, but in certain exemplary embodiments the protective member 98 is formed from a molded thermoplastic polyurethane elastomer, such as Pellethane^{™}.

The segments 104, 106 that form the multi-layer seal 96 and the protective member 98 can be held together using various techniques known in the art. As shown in FIG. 18, the segments 104, 106 can be held together by a plurality of ring members that mate to engage the segments 104, 106 therebetween. In particular, the protective member 98 can be engaged between the washer 86 and the gasket ring 88, and the seal 96 can be engaged between the gasket ring 88 and the retainer ring 90. Pins 108 on the crown 84, the gasket ring 88, and the retainer ring 90 can be used to mate the various members and to extend through and engage the segments 104, 106 of the seal 96 and the protective member 98. The crown 84 can be positioned above the washer 86 to help position the washer 86, which can generally be a flexible member configured to help provide a seal between the crown 84 and the gasket ring 88. The spacer seal 92 can be positioned between the retainer ring 90 and the first distal seal 94 to ensure an air and liquid tight seal between the first sealing port 22a and the seal base 16. The first sealing port 22a can also include the first distal seal 94, which can have a proximal flange that is captured between the spacer seal 92 and the seal base 16 to secure the first distal seal 94 therebetween. When fully assembled, the first port housing can be disposed at various locations within the surgical access device 10, e.g., in the first port opening 74a formed in the base 16.

In use, a surgical instrument can be passed through a center opening of the protective member 98 and the multi-layer seal 96, and the seal segments 104, 106 can engage and form a seal around an outer surface of the instrument to thereby prevent the passage of fluids and gas through the seal. When no instrument is disposed therethrough, the center opening will generally not form a seal in the working channel, however other configurations in which a seal is formed when no instrument is disposed therethrough are also conceivable. Exemplary instrument seal configurations are described in more detail in U.S. Patent Publication No. 2004/0230161 entitled "Trocar Seal Assembly," filed on March 31, 2004, and U.S. Patent Application No. 10/687,502 entitled "Conical Trocar Seal," filed on October 15, 2003, which are hereby incorporated by reference in their entireties. The instrument can be further inserted through the sealing element, e.g., through the first distal seal 94. When no instrument is disposed therethrough, the first distal seal 94 can be configured to form a seal in the working channel, however other configurations in which a seal is not formed without an instrument is disposed therethrough are also conceivable.

The second and third sealing ports 22b, 22c can generally be configured and used as a channel seal. In this illustrated embodiment, as shown in FIG. 19, the second and third sealing ports 22b, 22c can each include the deep cone 100 having a proximal flange 110 configured to seat on a proximal flange 112 of the second distal seal 102 with a distal portion 114 of the deep cone 100 configured to be disposed within the second distal seal 102. The second and third sealing ports 22b, 22c can be secured between the cap 14 and the seal base 16 with a proximal surface of the deep cone's proximal flange engaging the cap 14 and a distal surface of the second distal seal's proximal flange 112 engaging the base 16.

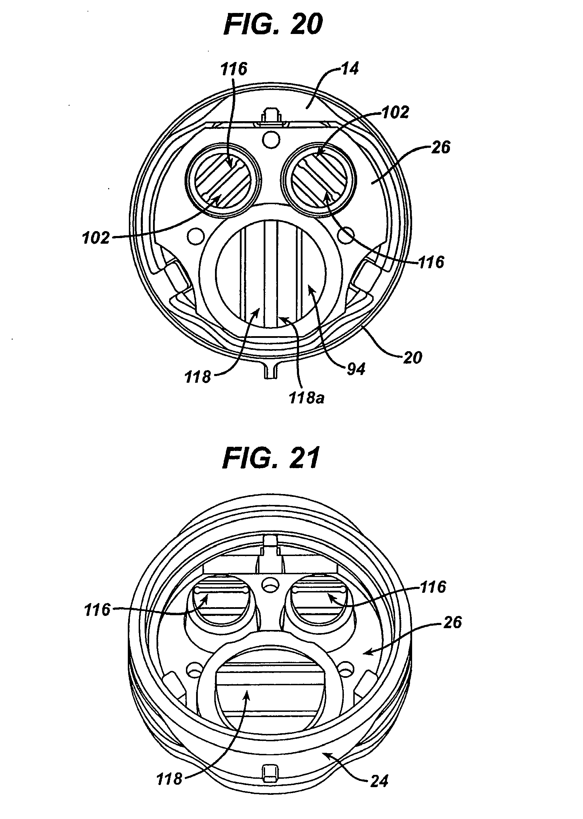

As shown in FIG. 12, the housing 12 and the proximal retractor base 24 can have a height H that is less than a longitudinal length of the device's sealing elements. In such a configuration, one or more of the sealing elements can be oriented to minimize contact with the retractor 18. For non-limiting example, as shown in FIG. 20, the second and third distal seals 102 can be aligned with their respective distal sealing element openings 116 (shown in a closed position) tangential to a perimeter of the housing 12 respectively closest to the second and third distal seals 102, and hence also to a perimeter of the working channel 18a of the retractor 18 extending from the housing 12 when the housing 12 is mated to the retractor 18. Such an alignment can help prevent the distal sealing element openings 116 from being pushed open by an inner wall of the retractor's inner elongate portion 32 when the seal base 16 is moved relative to the retractor 18. In contrast, the first distal seal 94 can be aligned with its distal sealing element opening 118 (shown in a closed position) perpendicular to the perimeter of the housing 12 that is located a shortest distance from the first distal seal 94, and hence also to the closest portion of the perimeter of the working channel 18a of the retractor 18 extending from the housing 12 when the housing 12 is mated to the retractor 18. A terminal end 118a of the distal sealing element opening 118 can be positioned to axially align with the perimeter of the housing 12 or to be positioned radially inward of the housing's perimeter to help prevent the retractor 18 from pushing open the distal sealing element opening 118. Such a perpendicular alignment for the first distal seal 94 can allow more range of motion for an instrument inserted therethrough because of the relatively large size of the first sealing port 22a seated in the first port opening 74a. An instrument inserted therethrough can also be more likely to push through the distal sealing element opening 118 without the first distal seal 94 being limited in movement by the inner elongate portion 32 of the retractor 18 as the opposed flaps defming the distal sealing element opening 118 move apart from one another. The distal seal openings 116, 118 are each linear, elongate slots in this illustrated embodiment, but as mentioned above, any of the distal seal openings 116, 118 can have a different shape. In some embodiments, the seal base 16 and/or the housing 12 can have a height H to accommodate a full length of the sealing elements to prevent the sealing elements from coming into contact with the interior of the retractor 18.

In another embodiment illustrated in FIG. 21, the sealing ports 22a, 22b, 22c can be seated in the seal base 26 such that the distal sealing element opening 118 of the first distal seal 94 and the distal sealing element openings 116 of the second and third distal seals 102 can each be tangential to the perimeter of the housing 12 respectively closest to the distal sealing element openings 116, 118, and hence also to the retractor 18 extending from the housing 12 when the housing 12 is mated thereto.

As mentioned above, the sealing ports 22a, 22b, 22c, including their respective port housings and respective sealing elements, can be configured to be in a fixed position relative to the base 16 and to rotate with the housing 12 relative to the retractor 18. However, any one or more of the sealing ports 22a, 22b, 22c can be configured to be movable relative to any one or more portions of the housing 12, such as the housing 12, the base 16, or any others of the sealing ports 22a, 22b, 22c.

Although the housing 12 can be configured to be movable relative to the retractor 18 with or without any instruments inserted through any of the sealing ports 22a, 22b, 22c, e.g., by being manually rotated by hand, the housing 12 can also be configured to move relative to the retractor 18 in response to motion of at least one instrument inserted through one of the ports 22a, 22b, 22c.

In use, one or more surgical instruments can be inserted into a body cavity through the surgical access device 10, which can help optimally position the surgical instruments relative to the body cavity through movement of the housing 12 relative to the retractor 18. The device 10 can be positioned within tissue to provide access to a body cavity underlying the tissue in a variety of ways. In one embodiment, the device 10 can be positioned in tissue fully assembled in the default position shown in FIGS. 1, 2A and 3. In another embodiment, the device 10 can be positioned partially assembled in tissue and be fully assembled with a portion of the device 10 positioned in the tissue.

In one embodiment, the retractor 18 can be positioned within an opening or incision formed in tissue, e.g., in the umbilicus, with the proximal and distal flanges 29, 30 of the retractor 18 positioned on opposed sides of the tissue. The proximal retractor base 24 in the proximal portion of the retractor 18 can be positioned on one side of the tissue with a distal surface of the proximal retractor base 24 positioned on and/or proximal to a proximal surface of the tissue. The distal flange 30 of the retractor 18 can be positioned on and/or distal to a distal surface of the tissue in a body cavity underlying the tissue. The inner elongate portion 32 of the retractor 18 can thereby be positioned within the tissue with the working channel 18a of the retractor 18 extending through the tissue to provide a path of access to the body cavity.

With the retractor 18 positioned in the tissue, the housing 12 can be attached to the retractor 18 to fully assemble the device 10. If the tissue and/or the retractor 18 are adequately flexible, the retractor 18 can be angled or pivoted to a desired position to ease attachment of the housing 12 to the retractor 18. The retractor 18 can also be angled or pivoted during use of the device 10 with one or more surgical instruments inserted therethrough. To mate the housing 12 to the retractor 18, the housing 12 can be positioned proximal to the retractor 18 with a distal portion of the housing 12 engaging a proximal portion of the retractor 18, e.g., the distal surface 60 of the distal housing 20 engaging the lip 44 of proximal retractor base 24. In embodiments where the base 16 and/or the distal housing 20 are not configured to lock to the retractor 18 without an engagement and release mechanism releasably locking the housing 12 to the retractor 18, e.g., if the base 16 and/or the distal housing 20 are not integrally formed with the cap 14, the base 16 and/or the distal housing 20 can be positioned proximal to the retractor 18 before the cap 14 is attached to the retractor 18. As mentioned above, the bayonet feet 46a, 46b, 46c of the housing 12 can be positioned in the openings 48a, 48b, 48c of the proximal retractor base 24, and the housing 12 can be rotated relative to the retractor 18 to lock the housing 12 thereto. The tissue can provide adequate tension such that the retractor 18 need not be held in position while the housing 12 is rotated relative thereto, although the retractor 18 can be so held to help provide support to the device 10 during its assembly.

With the surgical access device 10 assembled and positioned in the tissue, one or more surgical instruments can be inserted therethrough and into the body cavity where the instruments can help perform any type of surgical procedure.

At any point before, during, or after a surgical procedure, the housing 12 in full or part can be released from the retractor 18, and the retractor 18 can be removed from the tissue. To disengage the housing 12 from the retractor 18, the housing 12 can be rotated relative to the retractor 18 and/or the alignment mechanism can be engaged to indicate that the housing 12 and retractor 18 are in a predetermined rotational orientation to allow the housing 12 to be removed from the retractor 18. The engagement and release mechanism can then be disengaged, e.g., the housing 12 can be proximally moved to disengage the bayonet feet 46a, 46b, 46c from the openings 48a, 48b, 48c. The tissue can provide adequate tension for the proximal motion of the housing 12.

With the housing 12 of the device 10 disengaged from the retractor 18, the working channel 18a of the retractor 18 can still provide access to the body cavity underlying the tissue. One or more surgical instruments can be advanced through the working channel 18a, such as a waste removal bag configured to hold waste material, e.g., dissected tissue, excess fluid, etc., from the body cavity. The bag can be introduced into the body cavity through the retractor's working channel 18a or other access port. A person skilled in the art will appreciate that one or more surgical instruments can be advanced through the retractor's working channel 18a before and/or after the housing 12 has been attached to the retractor 18.

As surgical instruments are inserted through the surgical access device embodiments described herein, a risk can exist that a particularly sharp instrument may tear or puncture a portion of the retractor or nearby tissue. Accordingly, in any and all of the embodiments described herein, a safety shield can optionally be included to reduce the risk of tearing or puncture by a surgical instrument. In general the shield can be of a material that is relatively smooth and with a low coefficient of friction to allow ease of passage of instruments, but resistant to tearing and puncture. For example, the shield can be formed of silicone, urethane, thermoplastic elastomer, rubber, polyolefins, polyesters, nylons, fluoropolymers, and any other suitable materials known in the art. The shield can generally provide a liner for a retractor of tissue and can be detachable from a surgical access device so it can be used as needed in a particular procedure. Exemplary embodiments of safety shields are described in more detail in previously mentioned U.S. Patent Application No. 2006/0247673 entitled "Multi-port Laparoscopic Access Device" filed November 2, 2006 and in U.S. Application No. [ ] entitled "Methods and Devices for Providing Access to a Body Cavity" [Atty. Docket No. 100873-337 (END6551USNP)] filed on even date herewith and in U.S. Application No. 12/242,765 entitled "Surgical Access Device" filed on September 30, 2008, which are hereby incorporated by reference in their entireties.

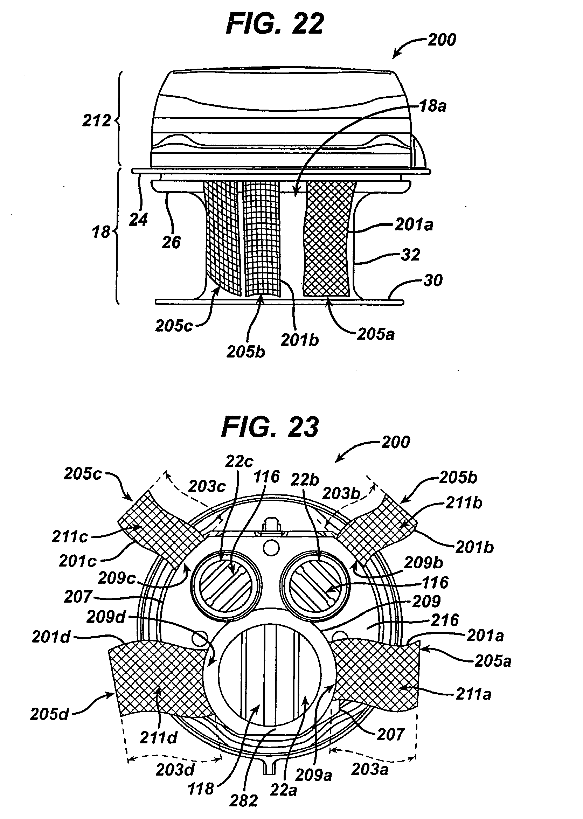

In one exemplary embodiment shown in FIGS. 22 and 23, a surgical access device 200 includes a safety shield in the form of a plurality of flexible safety strips. The surgical access device 200 can be configured and used similar to the surgical access device 10 discussed above and can include a housing 212, similar to the housing 12 discussed above, that is configured to be releasably mated to the retractor 18. The housing 212 can include a seal base 216, similar to the seal base 26 discussed above, and have the first, second, and third sealing ports 22a, 22b, 22c seated therein. The sealing ports 22a, 22b, 22c in this illustrated embodiment are fixed relative to the base 216, but as mentioned above, any one or more of the sealing ports 22a, 22b, 22c can be movable relative to the base 216. The sealing ports 22a, 22b, 22c can be aligned in any way relative to the housing 12 and to the retractor 18 when the housing 12 is mated thereto, such as shown in FIG. 23 similar to the sealing ports 22a, 22b, 22c in FIG. 20 with the first distal seal 94 aligned with its distal sealing element opening 118 (shown in a closed position) perpendicular to the perimeter of the housing 12 closest to the first distal seal 94 and the second and third distal seals 102 aligned with their respective distal sealing element openings 116 (shown in a closed position) tangential to the perimeter of the housing 12 that is respectively closest to the second and third distal seals 102.

Unlike the seal base 16 above, in this embodiment the seal base 216 has a plurality of flexible shields 201a, 20 1 b, 20 1 c, 201d attached thereto that are configured to extend through the retractor 18 when the housing 212 is mated thereto to thereby provide a protective lining as surgical instruments are inserted through the device 200 to help protect the retractor 18. The shields 201a, 201b, 201c, 201d can have any size, shape, and configuration, same or different from one another. Although the device 200 includes first, second, third, and fourth shields 201 a, 201 b, 201 c, 201 d, the device 200 can include any number of safety shields. In an exemplary embodiment, the device 200 has a quantity of safety shields equal to or greater than a quantity of sealing ports in the device 200 such that each sealing port has at least one associated shield. In this way, at least one safety shield 201a, 201b, 201c, 201d can be configured help protect the retractor 18, e.g., an inner wall of the inner elongate portion 32, from an instrument inserted through one of the sealing ports 22a, 22b, 22c when any number of instruments are inserted through the housing 212 into the working channel 18a. As illustrated in FIGS. 22 and 23, the first sealing port 22a, i.e., the larger diameter port, can have two associated shields 201 a, 201 d, and the second and third sealing ports 22b, 22c that are smaller than the first sealing port 22a can each have one associated shield 201b, 201c, respectively. The shields 201a, 201b, 201c, 201 d each have a substantially rectangular shape, although as mentioned above they can each have any shape, e.g., elliptical , trapezoidal, triangular, etc. Respective longitudinal lengths 203a, 203b, 203c, 203d of the shields 201a, 201b, 201c, 201d can have a length corresponding to a height of the retractor 18, but can also have lengths 203a, 203b, 203c, 203d less than or considerably longer than the height of the retractor 18 depending on a specific application. As in the embodiment illustrated in FIGS. 22 and 23 (the fourth shield 201d is obscured in FIG. 22), the lengths 203a, 203b, 203c, 203d of each of the shields 201a, 201b, 201c, 201d can be such that when the housing 212 is coupled to the retractor 18 the shields 201a, 201b, 201c, 201d are contained within the device 200 at least when the device 200 is in a default position, e.g., as shown in FIG. 22. The shields 201 a, 20 1 b, 201 c, 20 1 d can thereby extend distally through the working channel 18a of the retractor 18 with respective distal ends 205a, 205b, 205c, 205d of each of the shields 20 1 a, 201 b, 201c, 201 d being proximal to a distal end of the retractor 18, e.g., proximal to the distal flange 30, which can help prevent the shields 201a, 201 b, 201c, 201d from interfering with a surgical site inside a body cavity to which the device 200 provides access. The shields 201a, 201b, 201 c, 201 d can also each have any width, same or different from any of the other shields 201a, 201b, 201c, 201d. In an exemplary embodiment, the shields 201a, 201b, 201c, 201d can have a width that is equal to or greater than a width of their respective associated sealing ports 22a, 22b, 22c, e.g., the distal sealing element openings 116, 118 of the sealing ports 22a, 22b, 22c.

Being attached to the base 216, the shields 201a, 201b, 201c, 201d can thus each rotate relative to the retractor 18 along with the base 216 when the housing 212 is coupled to the retractor 18 and rotated relative thereto. A person skilled in the art will appreciate that the shields 201a, 201b, 201c, 201d can be attached to the base 216, and/or any other portion of the housing 212, using any attachment mechanism, e.g., adhesive, sealant, press fit, etc. Regardless of where on the housing 212 the shields 201 a, 201b, 201 c, 201 d are attached, in an exemplary embodiment, each of the shields 201a, 201b, 201c, 201d can be located adjacent to one of the access ports 22a, 22b, 22c seated in the base 216 to more effectively protect the retractor 18 from instruments inserted through the access ports 22a, 22b, 22c.

As illustrated, the shields 201a, 201b, 201c, 201d can be configured to engage a perimeter of the base 216. The second and third shields 201b, 201c, e.g., the shields associated with the smaller sealing ports 22b, 22c seated in raised housings (not shown) of the base 216, can be attached at their respective proximal ends 209b, 209c to an outer perimeter 207 of a proximal base surface (not shown) of the base 216. The first and fourth shields 201a, 201d, e.g., the shields associated with the larger sealing port 22a seated in a recessed portion 282 of the base 216 distal to the proximal base surface, can be attached at their respective proximal ends 209a, 209d to an outer perimeter 209 of the recessed portion 282. The shields 201a, 201b, 201c, 201d can thus be attached in different horizontal planes relative to the base 216, e.g., with the second and third shields 201b, 201c attached to the base 216 in a plane proximal to a plane in which the first and fourth 201a, 201d are attached to the base 216. The shields 201a, 201b, 201c, 201d can each be attached to the base 216 (and/or elsewhere on the housing 212) such that the distal sealing element openings 116, 118 of the various sealing ports 22a, 22b, 22c can each extend parallel to at least one planar surface 211 a, 211 b, 211 c, 211 d of one of the respective shields 201a, 201b, 201c, 201d. As shown in FIG. 22, the distal sealing elements openings 116 of the second and third sealing ports 22b, 22c can be configured to extend tangential to the perimeter of the working channel 18a in the retractor 18 when the housing 212 is coupled thereto and respectively extend parallel to the planar surfaces 211b, 211c of the second and third shields 201b, 201c. Similarly, the distal sealing element opening 118 of the first sealing port 22a can be configured to extend perpendicular to the closest portion of the perimeter of the working channel 18a in the retractor 18 when the housing 212 is coupled thereto and respectively extend parallel to the planar surfaces 211 a, 211d of the first and fourth shields 201a, 201d.

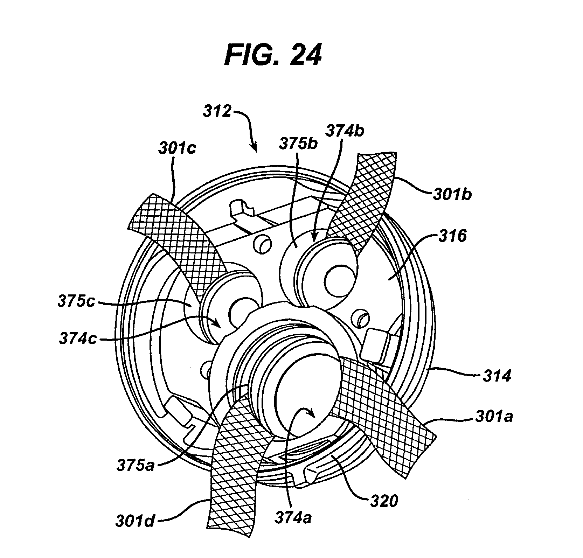

In another embodiment shown in FIG. 24, a housing 312 of a surgical access device can be configured to releasably mate to a retractor and can include a proximal housing or cap 314, a distal housing 320, and a seal base 316 to which a plurality of shields 301a, 301b, 301c, 301d can be attached. The shields 301a, 301b, 301c, 301d can be sized, shaped, configured, and used similar to the shields 201a, 201b, 201c, 201d of FIGS. 22 and 23, but in this embodiment, the shields 301a, 301b, 301c, 301d are attached to the base 316 on inner surfaces 375a, 375b, 375c of respective port openings 374a, 374b, 374c formed in the base 316. The shields 301a, 301b, 301c, 301d can thereby by positioned adjacent to sealing ports (not shown) seated in the base 316 and aligned relative to distal openings thereof as discussed above. The sealing ports seated in the port openings 374a, 374b, 374c can be configured to secure the shields 301a, 301b, 301c, 301d to the base 316 by interference fit, although one or more other attachment mechanisms can be used instead or in addition. The shields 301a, 301b, 301c, 301d in this embodiment are each positioned a shorter distance from their associated sealing ports seated in the base 316 than the shields 201 a, 201b, 201c, 201d of FIGS. 22 and 23, which can improve chances of instruments inserted through the housing 312 into the retractor 18 contacting at least one of the shields 301 a, 301b, 301c, 301d before contacting an inner wall of the retractor 18, which can help better protect the retractor 18 from damage by the instruments.

As will be appreciated by those skilled in the art, any and all of the embodiments disclosed herein can be interchangeable with one another as needed. For example, an exemplary surgical access device kit could include multiple housings with one or more retractors. Each housing can have different sealing port configurations including different types of sealing ports, different numbers of sealing ports, etc. as needed in particular application. Various release mechanism known in the art can be used to releasably attach the various housings to a retractor.

There are various features that can optionally be included with any and all of the surgical access device embodiments disclosed herein. For example, a component of the device, such as a seal cap, retractor, etc., can have one or more lights formed thereon or around a circumference thereof to enable better visualization when inserted within a patient. As will be appreciated, any wavelength of light can be used for various applications, whether visible or invisible. Any number of ports can also be included on and/or through the surgical access devices to enable the use of various surgical techniques and devices as needed in a particular procedure. For example, openings and ports can allow for the introduction of pressurized gases, vacuum systems, energy sources such as radiofrequency and ultrasound, irrigation, imaging, etc. As will be appreciated by those skilled in the art, any of these techniques and devices can be removably attachable to the surgical access device and can be exchanged and manipulated as needed.

The embodiments described herein can be used in any known and future surgical procedures and methods, as will be appreciated by those skilled in the art. For example, any of the embodiments described herein can be used in performing a sleeve gastrectomy and/or a gastroplasty, as described in U.S. Application No. 12/242,765 entitled "Surgical Access Device" filed on September 30, 2008; U.S. Application No. 12/242,711 entitled "Surgical Access Device with Protective Element" filed on September 30, 2008; U.S. Application No. 12/242,721 entitled "Multiple Port Surgical Access Device" filed on September 30, 2008; U.S. Application No. 12/242,726 entitled "Variable Surgical Access Device" filed on September 30, 2008; U.S. Application No. 12/242,333 entitled "Methods and Devices for Performing Gastrectomies and Gastroplasties" filed on September 30, 2008; U.S. Application No. 12/242,353 entitled "Methods and Devices for Performing Gastrectomies and Gastroplasties" filed on September 30, 2008; and U.S. Application No. 12/242,381 entitled "Methods and Devices for Performing Gastroplasties Using a Multiple Port Access Device" filed on September 30, 2008, all of which are hereby incorporated by reference in their entireties.

The devices disclosed herein can be designed to be disposed of after a single use, or they can be designed to be used multiple times. In either case, however, the device can be reconditioned for reuse after at least one use. Reconditioning can include any combination of the steps of disassembly of the device, followed by cleaning or replacement of particular pieces, and subsequent reassembly. In particular, the device can be disassembled, and any number of the particular pieces or parts of the device can be selectively replaced or removed in any combination, e.g., a seal base, a proximal retractor base, a seal cap, etc. Upon cleaning and/or replacement of particular parts, the device can be reassembled for subsequent use either at a reconditioning facility, or by a surgical team immediately prior to a surgical procedure. Those skilled in the art will appreciate that reconditioning of a device can utilize a variety of techniques for disassembly, cleaning/replacement, and reassembly. Use of such techniques, and the resulting reconditioned device, are all within the scope of the present application.

Preferably, the invention described herein will be processed before surgery. First, a new or used instrument is obtained and if necessary cleaned. The instrument can then be sterilized. In one sterilization technique, the instrument is placed in a closed and sealed container, such as a plastic or TYVEK bag. The container and instrument are then placed in a field of radiation that can penetrate the container, such as gamma radiation, x-rays, or high-energy electrons. The radiation kills bacteria on the instrument and in the container. The sterilized instrument can then be stored in the sterile container. The sealed container keeps the instrument sterile until it is opened in the medical facility.

It is preferred that device is sterilized. This can be done by any number of ways known to those skilled in the art including beta or gamma radiation, ethylene oxide, steam, and a liquid bath (e.g., cold soak).

One skilled in the art will appreciate further features and advantages of the invention based on the above-described embodiments. Accordingly, the invention is not to be limited by what has been particularly shown and described, except as indicated by the appended claims. All publications and references cited herein are expressly incorporated herein by reference in their entirety.

What is claimed is:

## Claims

1. A surgical access device, comprising:
a retractor having a working channel extending therethrough for forming a pathway through tissue into a body cavity; and
a housing having a plurality of sealing ports configured to receive an instrument therethrough and into the working channel, the housing being configured to releasably mate to the retractor in only one predetermined rotational orientation.

2. The device of claim 1, wherein the housing is configured to rotate relative to the retractor from an initial mating position, in which the housing is freely releasable from the retractor, to a second rotated position, in which the housing is prevented from disengagement from the retractor.

3. The device of claim 1, further comprising an alignment mechanism formed on at least one of the housing and the retractor and configured to indicate alignment of the housing and the retractor in the predetermined rotational orientation.

4. The device of claim 3, wherein the alignment mechanism comprises a first protrusion extending laterally outward from a sidewall of the housing and configured to align with a second protrusion extending laterally outward from a sidewall of the retractor.

5. The device of claim 1, wherein the housing has a plurality of mating elements that are configured to releasably engage the retractor to mate the housing to the retractor, and wherein at least one of the plurality of mating elements differs from another one of the plurality of mating elements.

6. The device of claim 5, wherein the mating elements rotatably mate the housing to the retractor.

7. The device of claim 5, wherein the plurality of mating elements comprise a plurality of feet extending distally from the housing and configured to engage a plurality of proximally-facing openings formed in the retractor.

8. The device of claim 7, wherein a proximal portion of the retractor has the plurality of proximally-facing openings formed therein, and wherein the plurality of feet are configured to engage the proximal portion of the retractor when the housing is rotated relative to the retractor from the predetermined rotational orientation.

9. The device of claim 7, wherein at least one of the plurality of proximally-facing openings formed in the retractor has a longitudinal length that differs from at least one other of the plurality of proximally-facing openings.

10. The device of claim 7, wherein each of the plurality of proximally-facing openings formed in the retractor has a size that differs from one another, and each of the plurality of feet has a size corresponding to one of the plurality of openings.

11. The device of claim 1, further comprising a flexible shield disposed within the retractor when the housing is mated to the retractor, the flexible shield being configured to protect the retractor from damage caused by insertion of a surgical instrument through the retractor.

12. The device of claim 11, wherein the flexible shield comprises a plurality of flexible strips extending distally from the housing.

13. The device of claim 1, wherein each sealing port comprises an opening formed through the housing and having at least one sealing element disposed therein, the at least one sealing element being configured to form at least one of a seal around an instrument inserted therethrough and a channel seal configured to form a seal when no instrument is inserted therethrough.

14. A surgical access device, comprising:
a retractor having an opening extending therethrough for forming a pathway through tissue into a body cavity; and
a housing rotatably coupled to the retractor and having a plurality of sealing ports in communication with the opening in the retractor, each of the sealing ports having a sealing element fixedly disposed therein, at least one of the sealing elements having a linear distal opening extending tangential to a perimeter of the opening in the retractor and at least one other of the sealing elements having a linear distal opening extending perpendicular to the perimeter of the opening in the retractor.

15. A surgical access device, comprising:
a proximal portion having a plurality of access ports formed therein for receiving surgical instruments therethrough;
a distal portion removably coupled to the proximal portion and having an opening extending therethrough for forming a pathway through tissue into a body cavity; and
a plurality of flexible shields extending from the proximal portion and configured to extend into the opening of the distal portion, each of the flexible shields being located adjacent to one of the access ports.
